Europäisches Patentamt

European Patent Office

Office européen des brevets

(11) Veröffentlichungsnummer: **0 163 979 B2**

# (12) NEUE EUROPÄISCHE PATENTSCHRIFT

(45) Veröffentlichungstag der neuen Patentschrift:
18.12.91 Patentblatt 91/51

(21) Anmeldenummer: 85105730.7

(22) Anmeldetag: 09.05.85

(51) Int. Cl.⁵: **A61F 5/44**

(54) **Anus-praeter-Versorgungssystem.**

(30) Priorität: 09.05.84 DE 3417183

(43) Veröffentlichungstag der Anmeldung:
11.12.85 Patentblatt 85/50

(45) Bekanntmachung des Hinweises auf die
Patenterteilung:
02.11.88 Patentblatt 88/44

(45) Bekanntmachung des Hinweises auf die
Entscheidung über den Einspruch:
18.12.91 Patentblatt 91/51

(84) Benannte Vertragsstaaten:
DE FR GB NL SE

(56) Entgegenhaltungen:
EP-A- 0 089 138
DE-A- 2 812 833

(56) Entgegenhaltungen:
DE-A- 3 417 183
GB-A- 27 254
GB-A- 1 021 145
GB-A- 1 568 860
GB-A- 1 579 875
US-A- 3 100 488
US-A- 3 827 435
US-A- 4 256 110
New England Journal of Medicine

(73) Patentinhaber: Hauer, Gerald Dr.
Sintzenichstrasse 2a
W-8000 München 71 (DE)

(72) Erfinder: Hauer, Gerald Dr.
Sintzenichstrasse 2a
W-8000 München 71 (DE)

(74) Vertreter: Patentanwälte Dr. Solf & Zapf
Zeppelinstrasse 53
W-8000 München 80 (DE)

EP 0 163 979 B2

## Beschreibung

Die Erfindung betrifft ein Anus-praeter-Versorgungssystem mit einer Basisplatte, die einen eine Ausnehmung umschließenden Anschlußring aufweist, und mit einem mit dem Anschlußring in Eingriff bringbaren, eine Öffnung in einem Beutel umschließenden Rastring, wobei der Anschlußring und der Rastring jeweis mindestens einen, sich im wesentlichen in einer Ebene senkrecht zur Drehachse der beiden Ringe und über einen bestimmten Winkelbereich erstreckenden Vorsprung aufweisen und in eine sich hintergreifende Lage gegeneinander verdrehbar sind.

Es sind bereits derartige Anus-praeter-Versorgungssysteme bekannt, bei denen zur Verstärkung von Stomata und Fisteln an geeigneter Stelle auf den Körper des patienten eine Basisplatte aufgeklebt wird. Die Basisplatte weist in ihrem mittleren Bereich eine Ausnehmung auf, die auf der dem Körper des Patienten abgewandten Seite von einem Rastring umgeben ist. Das Versorgungssystem umfaßt außerdem einen Beutel, der eine Öffnung aufweist, die von einem zweiten Rastring vollständig umschlossen ist. Die beiden Rastringe sind so ausgestaltet, daß sie in ihrem Querschnitt ein nut- oder federähnliches Profil zeigen, so daß sich die beiden Rastringe in der Art eines Druckknopfes miteinander in Eingriff bringen lassen. Auf diese Weise erfolgt eine dichte und feste Verbindung des Beutels mit der Basisplatte.

Bei dieser Ausgestaltung des Anus-praeter-Versorgungssystems kann die Basisplatte über einen längeren Zeitraum am Körper des Patienten verbleiben, während der Beutel zur Entleerung oder zum Austausch gegen einen anderen Beutel mehrfach abgenommen werden kann. Sowohl zum Lösen der beiden Rastringe voneinander bei der Abnahme des Beutels als auch zum Anbringen eines neuen Beutels ist es bei einem solchen System notwendig, eine Kraft auf den Körper des Patienten einwirken zu lassen, sei es eine Gegenhaltekraft beim Abziehen des Beutels oder eine direkte Druckkraft beim Anschließen eines Beutels. Diese Krafteinwirkung auf den Körper des Patienten führt zu erheblichen Schmerzen, insbesondere bei frisch operierten Patienten.

Aus der Furcht, derartige Schmerzen hervorzurufen, werden die Rastringe oftmals nicht fest genug aneinander gedrückt, was zur Folge hat, daß das System undicht wird. Eine Undichtigkeit eines derartigen Systems führt nicht nur zu einer erheblichen Geruchsbelästigung, sondern ist auch wegen des Auftretens von Keimen in höchstem Maße unhygienisch.

Andere bekannte Anus-pater-Versorgungssysteme weisen einen fest mit der Basisplatte verbundenen Beutel auf, der an seinem unteren Ende eine Entleerungsöffnung aufweist. Diese Beutel können zwar über einen längeren Zeitraum verwendet werden, sie müssen jedoch in regelmäßigen Abständen durch die Entleerungsöffnung entleert werden. Dies ist sowohl für den Patienten als auch für das Pflegepersonal eine sehr unangenehme Angelegenheit. Bei diesen Systemen ist es ferner erforderlich, die Entleerungsöffnung des Beutels in entsprechender Weise dicht zu verschließen, was einen erheblichen Aufwand erfordert und nicht immer mit der entsprechenden Sicherheit durchgeführt werden kann.

Es sind auch Anus-praeter-Versorgungssysteme bekannt, bei denen der Beutel, der mit der Basisplatte fest verbunden ist, keine Entleerungsöffnung aufweist, sondern nach jeder Füllung mitsamt der Basisplatte gegen ein komplettes neues Versorgungssystem ausgetauscht werden muß. Das häufige Wechseln der Basisplatte verursacht dem Patienten ebenfalls starke Schmerzen und ist für ihn ausgesprochen unangenehm. Andererseits erhöhen sich auch die Kosten eines derartigen Systems ganz beträchtlich, da stets die komplette Basisplatte mit ausgetauscht werden muß.

Aus der GB-A-1 579 875 ist ebenfalls ein Anus-praeter-Versorgungssystem mit einer Basisplatte bekannt, die am Körper des Patienten befestigt werden kann und eine Kupplung aus einem ersten und einem zweiten Kupplungselement aus Kunststoffmaterial aufweist, die auf mechanische Weise in Art eines Bajonettverschlusses aneinander befestigt werden können mit einem Abdichtring dazwischen, wobei an dem zweiten Kupplungselement ein Beutel befestigt sein kann. Die bei diesem bekannten Anus-praeter-System zwisen den beiden Ringen vorgesehene Konusdichtung hat jedoch die vorstehend geschilderten Nachteile.

Aufgabe der Erfindung war es daher, ein Anus-praeter-Versorgungssystem der eingangs geschilderten Art zu schaffen, das bei einfachem Aufbau und einfacher Bedienung einen sicheren und für den Patienten schmerzfreien Wechsel des Beutels ermöglicht.

Es wurde nun gefunden, daß diese Aufgabe bei einem Anuspraeter-Versorgungssystem mit einer Basisplatte, die einen eine Ausnehmung umschließenden Anschlußring aufweist, und mit einem mit dem Anschlußring in Eingriff bringbaren, eine Öffnung in einem Beutel umschließenden Rastring, wobei der Anschlußring und der Rastring jeweils mindestens einen, sich im wesentlichen in einer Ebenen senkrecht zur Drehachse der beiden Ringe und über einen bestimmten Winkelbereich erstreckenden Vorsprung aufweisen und in eine sich hintergreifende Lage gegeneinander verdrehbar sind, erfindungsgemäß dadurch gelöst werden kann, daß im Rastring ein zylindrischer, mit dem Beutel verbundener Teil drehbar gelagert ist, der an seiner Zylinderinnenfläche eine axial-radiale Ring-Dichtlippe aufweist, welche die Zylinderaußenfläche des Anschlußringes dich-

2

tend umgreift.

Gemäß einer bevorzugten Ausgestaltung der Erfindung sind die an beiden Ringen vorgesehenen Vorsprünge als Segmente konzentrischer Kreisringe ausgebildet oder sie bilden den Teil eines Schraubgewindes.

Gemäß einer weiteren bevorzugten Ausgestaltung der Erfindung weist der Beutel koaxial zum Rastring auf seiner Rückseite eine Öffnung auf, deren Rand konisch ausgebildet ist zur Aufnahme eines Irrigatorschlauches.

Das erfindungsgemäße Anus-praeter-Versorgungssystem weist gegenüber dem Stand der Technik wesentliche Vorteile auf. Da die Basisplatte bei einem Wechsel des Beutels nicht mit ausgetauscht zu werden braucht, ergeben sich sowohl in finanzieller Hinsicht erhebliche Vorteile als auch für den Patienten, dem die schmerzbereitende Abnahme und Anbringung einer Basisplatte erspart bleibt. Durch die erfindungsgemäße Ausgestaltung des Anschlußbrings und des Rastrings ist gewährleistet, daß der Beutel beliebig oft von der Basisplatte gelöst bzw. wieder mit dieser verbunden werden kann, ohne daß Druckkräfte aufgebracht werden müssen, welche gegen den Körper des Patienten gerichtet sind. Ein Lösen des Beutels ist deshalb für den Patienten völlig schmerzfrei. Die drehbare Ausgestaltung der Ringe ermöglicht, die Verbindung fast ohne Krafteinwirkung zu lösen bzw. herzustellen. Weiterhin ergibt sich durch die erfindungsgemäße Ausgestaltung der Vorsprünge eine hervorragende Belastbarkeit der Verbindung in axialer Richtung, so daß sichergestellt ist, daß auch in einem Notfall der Inhalt Beutels nicht austreten kann. Da eine Betätigung der Verbindungseinrichtung praktisch ohne Krafteinwirkung erfolgt, ist es möglich, eine wesentlich kleiner dimensionierte Basisplatte zu verwenden bzw. die Basisplatte durch ein entsprechend ausgestaltes Heftpflaster zu ersetzen.

Mit Vorteil ist das erfindungsgemäße Versorgungssystem so ausgestaltet, daß die Vorsprünge als Segmente konzentrischer Kreisringe ausgebildet sind. Diese Ausgestaltung ermöglicht eine besonders einfache und preisgünstige Herstellung des Anschlußrings und des Rastrings. Außerdem wird durch diese Ausgestaltung vermieden, daß es zu Klemmungen zwischen dem Anschlußring und dem Rastring bei deren Verdrehung kommt.

Eine vorteilhaft Ausgestaltung des erfindungsgemäßen Versorgungssystem ist auch dadurch gegeben, daß an einem Ende mindestens eines Vorsprungs ein die Drehbewegung begrenzender Anschlag vorgesehen ist. Bei dieser Ausgestaltung ist der maximale Verdrehbereich der beiden Ringe gegeneinander in sicherer Weise begrenzt, so daß vermieden wird, daß durch eine zu starke Drehung wiederum ein Lösen beiden Ringe voneinander erfolgen kann. Die Vorsprünge können vorzugsweise auch leicht geneigt angeordnet sein oder an einem Ende mit einer leicht ansteigenden Erhöhung versehen sein, so daß eine zusätzliche Klemmwirkung in der Endposition der Einrastung erreicht wird.

Eine günstige Ausgestaltung des erfindungsgemäßen Versorgungssystems ist auch dadurch gegebeb, daß mehrere Vorsprünge vorgesehen sind und daß die Vorsprünge und die Zwischenräume jeweils einen gleichen Kreissektor der Ringe umfassen. Bei dieser Ausgestaltung erfolgt stets eine optimale Zuordnung des Rastrings zu dem Anschlußring, gleichgültig in welcher Drehlage das den Anschlußring tragende Basisteil auf den Körper des Patienten aufgeklebt wurde. Außerdem erhöht sich die mechanische Festigkeit der verbindung zwischen dem Anschlußring und dem Rastring.

Mit Vorteil ist das erfindungsgemäße versorgungssystem auch so ausgestaltet, daß die Vorsprünge als Teil eines Schraubgewindes ausgestaltet sind. Diese Ausgestaltung beinhaltet nicht nur eine besonders einfache und auch für eine ungelernte Pflegeperson problemlose Verwendung des erfindungsgemäßen Anus-praeter-Versotrgungssystems, sondern gibt auch die Möglichkeit, auf eine zusätliche Dichtung zwischen dem Anschlußring und dem Rastring zu verzichten, da diese beiden durch die Steigung des Gewindes gegeneinander in Anlage gebracht werden können.

Um eine wirkungsvolle flüssigkeits- und gasdichte Abdichtung zwischen dem Anschlußring und dem Rastring zu ermöglichen, ist das Versorgungssystem so ausgestaltet, daß im Bereich zwischen den Ringen eine ringförmige Dichtung vorgesehen ist.

Eine besonders günstige Ausbildung des erfindungsgemäßen Anus-praeter-versorgungssystems ist auch dadurch gegeben, daß der Beutel koaxial zum Rastring auf seiner Rückseite eine Öffnung aufweist, deren Rand zur Aufnahme eines Irrigatorschlauchs konisch ausgebildet ist. Durch diese Ausgestaltung ist es auf besonders einfache Weise möglich, eine Spülung mittels des Irrigatorschlauchs vorzunehmen, während der Beutel mit der Basisplatte verbunden ist, so daß das abfließende Wasser problemlos und ohne zu einer verschmutzung der Umgebung zu führen, in dem Beutel gesammelt werden kann. Auf diese Weise kann der Spülvorgang unter sehr einfachen und optimal hygienischen Bedingungen vorgenommen werden. Der Beutel ist mit einem konusförmig ausgebildeten Öffnungsring versehen, der mit einer elastischen Dichtung nach außen abgeschlossen ist. Durch die Dichtung ist der Irrigatorschlauch hindurchstreckbar.

Die besonders bevorzugte Ausführungsform der Erfindung besteht aus drei Einheiten. Die Basisplatte ist mit dem Anschlußring einstückig ausgebildet, der Beutel ist mit einem Zylinderring mit Ringdichtlippe einstückig ausgebildet, und ein Drehteil bildet die dritte Einheit. Der Anschlußring an der Basisplatte bildet mit dem Drehteil eine Art Schraubverschluß, wobei die Dichtungswirkung über die Ringdichtlippe und den Anschlußring erzeugt wird, ohne dabei die Verschwenkbarkeit des Beutels zu beeinträchtigen. Hierdurch ergibt sich eine leichtere Handhabung bei zuverlässiger Abdichtung.

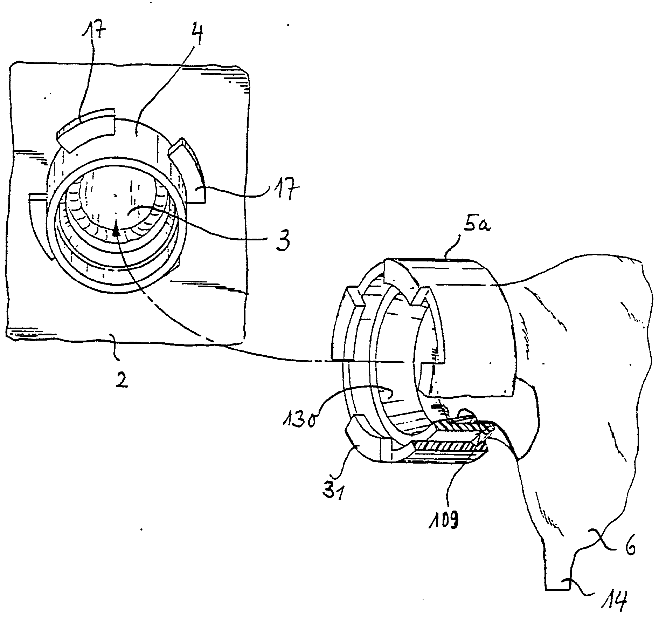

Nachstehend wird das erfindungsgemäße Anus-praeter-Versorgungssystem anhand eines bevorzugten Ausführungsbeispiels in Verbindung mit der beiliegenden Zeichnung näher beschrieben.

Das in der Zeichnung dargestellte Anus-praeter-Versorgungssystem zeigt eine auf den Körper des Patienten aufgeklebte Basisplatte 2, die in ihrem mittleren Bereich eine Ausnehmung 3 aufweist, die in geeigneter Weise an die Größe der Öffnung des Körpers des Patienten angepaßt wurde und in etwa koaxial zu dieser liegt. Bei dem dargestellten Ausführungsbeispiel weist die Ausnehmung 3 der Basisplatte 2 dieselbe Dimension auf wie die Öffnung im Körper des Patienten. Mit der Basisplatte ist ein Anschlußring 4 derart fest verbunden, daß die Ausnehmung 3 der Basisplatte 2 von dem Anschlußring umgeben wird. Die Verbindung des Anschlußrings 4 mit der Basisplatte 2 erfolgt durch ein geeignetes Klebe- oder Fügeverfahren.

Die Zeichnung zeigt ferner einen mit dem Anschlußring 4 in Eingriff bringbaren Rastring 5a, dessen innere Öffnung um einen gewissen Betrag größer ist als die Ausnehmung 3 in der Basisplatte 2. Mit dem Rastring 5a ist ein Beutel 6 fest verbunden. Der Beutel weist eine Öffnung auf, welche dieselben Dimensionen hat wie die Öffnung des Rastrings 5a und mit dieser koaxial angeordnet ist. Der Beutel 6 kann an seinem unteren Ende wahlweise eine Entleerungsöffnung 14 aufweisen, er kann aber auch an seinem unteren Ende geschlossen sein.

Der am Beutel 6 befestigte Rastring 5a weist einen inneren zylindrischen Teil 130 auf, von dem eine Ring-Dichtlippe 109 nach innen vorsteht, so daß durch Ineinanderfügen des Rastrings 5a in den Anschlußring 4 eine Abdichtungwirkung erzielt wird. An seinem nach außen gerichteten Bereich weist der Rastring 5a mehrere Vorsprünge 31 auf, die unter die vom äußeren Rand des Anschlußrings 4 nach innen weisenden Vorsprünge 17 des Anschlußrings 4 zur Anlage kommen. Auf diese Weise erfolgt eine sichere und stabile mechanische Verbindung zwischen dem Anschlußring 4 und dem Rastring 5a und damit zwischen dem Beutel 6 und dem Körper des Patienten.

Koaxial zur Öffnung des Rastrings 5a kann auf der gegenüberliegenden Seite des Beutels 6 eine Öffnung in Form eines konusförmigen Ringes vorgesehen sein (in der Zeichnung nicht dargestellt), der nach außen mit einer elastischen Dichtung abgeschlossen ist, durch die ein Irrigatorschlauch hindurchsteckbar ist. Der Irrigatorschlauch ist am vorderen Ende mit einer seitlichen Austrittsöffnung für den Austritt der Spüllösung für die Darmspülung ausgerüstet. Wenn die empfohlene Menge Spüllösung in den Darm eingelaufen ist, wird der Konusring aus dem Stoma entfernt und der Irrigatorschlauch aus der Dichtung, die sich danach selbständig schließt, herausgezogen.

Das in der Zeichnung teilweise in der Draufsicht dargestellte Ausführungsbeispiel des Anschlußrings 4 und des Rastrings 5a zeigt bei beiden Ringen jeweils vier (z. T. verdeckte) Vorsprünge 17 und 31. Die beiden Ringe sind punktsymmetrisch aufgebaut und weisen jeweils gleiche Breiten der Vorsprünge 17 und 31 bzw. der Zwischenräume zwischen den Vorsprüngen auf. Durch diese Ausgestaltung ist sichergestellt, daß die beiden Ringe miteinander in Eingriff gebracht werden können, ohne daß eine bestimmte Zuordnung zueinander vorliegen muß.

Es kann vorzugsweise jeweils an einem Ende jedes Vorsprungs 31 des Rastrings 5a ein Anschlag vorgesehen sein, der nach dem Einsetzen des Rastrings 5a in den Anschlußring 4 die maximale Drehbewegung begrenzt. Die Anschläge können durch Hochbiegen des Endbereiches der Vorsprünge 17 oder durch separat angeformte Anschläge gebildet sein. Bei dem dargestellten Ausführungsbeispiel sind die Vorsprünge 17 und 31 als Teile konzentrisch zueinanderliegender Kreisringe ausgestaltet.

Bei dem dargestellten Ausführungsbeispiel kann der Anschlußring 4 auch einen zylindrischen Teil aufweisen, an dessen Außenseite der Vorsprung 17 als Teil eines Schraubgewindes ausgeführt ist. Der dazugehörige Rastring 5a weist dann ebenfalls einen zylindrischen Bereich auf, der an seiner Innenseite entsprechende Teile eines Schraubgewindes trägt, so daß der Anschlußring 5a und der Rastring 4 mittels der miteinander in Eingriff stehenden Teile des Schraubgewindes in an sich bekannter Weise gegeneinander verschraubt werden können.

Die dargestellte Ausführungsform des erfindungsgemäßen Anus-praeter-Versorgungssystems weist im übrigen vorzugsweise die folgenden Besonderheiten auf:

Die Basisplatte 2 kann einstückig mit dem Anschlußring 4 ausgebildet sein. Der Anschlußring 4 steht von der Basisplatte 2 vor und weist auf seiner äußeren Mantelfläche unterbrochene Schraubgewindeteile 17 auf.

Der Rastring 5a, der am Beutel 6 befestigt ist, weist auf seiner Innenseite einen zylindrischen Teil 130 auf, von dem eine Ring-Dichtlippe 109 nach innen vorsteht, die in radialer Richtung federnd nachgiebig ist. Durch das Ineinanderfügen des Rastrings 5a in den Anschlußring 4 kann eine technisch einfache, aber hinsichtlich ihrer Abdichtungswirkung sehr wirkungsvolle Verbindung zwischen dem Beutel 6 und dem Körper des Patienten erzielt werden. Die beiliegenden Zeichnung läßt erkennen, wie der Beutel 16 mit dem Rastring 5a verbun-

EP 0 163 979 B2

den ist. Wesentlich ist dabei, daß der mit dem Beutel 6 fest verbundene Rastring 5a innen über die Lippendichtung 109 abgedichtet ist. Das ermöglicht, die Basisplatte innen zu erhöhen, so daß die Verbindung zwischen Anschlußring 4 und der Basisplatte 2 verbessert wird. Ein zusätzlicher Kunststoffüberzug auf der Basisplatte 2 kann damit entfallen.

## Patentansprüche

1. Anus-praeter-Versorgungssystem mit einer Basisplatte (2), die einen eine Ausnehmung umschließenden Anschlußring (4) aufweist, und mit einem mit dem Anschlußring in Eingriff bringbaren, eine Öffnung in einem Beutel (6) umschließenden Rastring (5a), wobei der Anschlußring und der Rastring jeweils mindestens einen, sich im wesentlichen in einer Ebene senkrecht zur Drehachse der beiden Ringe und über einen bestimmten Winkelbereich erstreckenden Vorsprung (17) aufweisen und in eine sich hintergreifende Lage gegeneinander verdrehbar sind, dadurch gekennzeichnet, daß im Rastring (5a) ein zylindrischer, mit dem Beutel (6) verbundener Teil (130) drehbar gelagert ist, der an seiner Zylinderinnenfläche eine axial-radiale Ring-Dichtlippe (109) aufweist, welche die Zylinderaußenfläche des Anschlußringes (4) dichtend umgreift.

2. Anus-praeter-Versorgungssystem nach Anspruch 1, dadurch gekennzeichnet, daß die an beiden Ringen (4, 5a) vorgesehenen Vorsprünge (17) als Segmente konzentrischer Kreisringe ausgebildet sind.

3. Anus-praeter-Versorgungssystem nach Anspruch 1, dadurch gekennzeichnet, daß die an den beiden Ringen (4, 5a) vorgesehenen Vorsprünge als Teil eines Schraubgewindes ausgestaltet sind.

4. Anus-praeter-Versorgungssystem nach einem der Ansprüche 1 bis 3, dadurch gekennzeichnet, daß der Beutel (6) koaxial zum Rastring (5a) auf seiner Rückseite eine Öffnung aufweist, deren Rand konisch ausgebildet ist zur Aufnahme eines Irrigatorschlauches.

## Claims

1. Anal prosthesis system with a baseplate (2) provided with a coupling ring (4) round a recess and with a lock ring (5a) round the mouth of the bag (6), which engages with the coupling ring, the coupling ring and lock ring each having at least one protrusion (17), essentially in a plane at right angles to their axis of rotation and extending through a set angle, and on turning the rings in relation to each other the protrusions engage behind each other characterised in that the locking ring (5a) contains a cylindrical part which is connected to the bag (130) and which can he rotated, which has an axial radial ring sealing lip (109) on the internal cylinder surface, which provides a seal around the outer cylinder surface of the coupling ring (4).

2. Anal prosthesis system according to Claim 1, characterised in that the protrusions ( 17) fitted to the two rings (4, 5a) are segments of concentric circular rings.

3. Anal prosthesis system according to Claim 1, characterised in that the protrusions fitted to the two rings (4, 5a) are part of a screw thread.

4. Anal prosthesis system according to one of Claims 1 to 3, characterised in that the bag (6) has an opening at the rear, concentric with the locking ring (5a), the edge of which is conical to accept a drain tube.

## Revendications

1. Système de soins par anus contre nature comprenant une plaque de base (2) munie d'une bague de connexion (4) entourant un évidement et une bague d'arrêt (5a) pouvant coopérer avec la bague de connexion et entourant une ouverture d'un sac, la bague de connexion et la bague d'arrêt présentant respectivement au moins une projection (17) s'étendant sensiblement dans un plan perpendiculaire à l'axe de rotation des deux bagues et sur une région angulaire prédéterminée, les deux bagues étant en outre pivotables l'une par rapport à l'autre dans une position où l'une entoure l'autre par l'arrière, caractérisé en ce qu'un élément (130) cylindrique, relié au sac (6), est disposé rotatif dans la bague d'arrêt (5a), cet élément présentant, sur sa face cylindrique intérieure une lèvre d'étanchéité annulaire (109) axiale et radiale qui entoure hermétiquement la face cylindrique extérieure de la bague de connexion (4).

2. Système de soins par anus contre nature selon la revendication 1, caractérisé en ce que les projections (17) prévues sur les deux bagues (4, 5a) ont la forme de segments de bagues annulaires concentriques.

3. Système de soins par anus contre nature selon la revendication 1, caractérisé en ce que les projections prévues sur les deux bagues (4, 5a) ont la forme d'une partie de filet de vis.

4. Système de soins par anus contre nature selon l'une des revendications 1 à 3, caractérisé en ce que le sac (6) présente sur sa face arrière et coaxialement à la bague d'arrêt (5a), une ouverture dont le bord est conique pour recevoir un tuyau d'irrigation.